# FASCICULE DE BREVET EUROPEEN

(11) **EP 3 057 542 B1**
(45) Date de publication et mention de la délivrance du brevet: **13.12.2017**
(21) Numéro de dépôt: 14796230.2
(22) Date de dépôt: 14.10.2014
(51) Int. Cl.: A61F 5/00

(54) **BANDE GASTRIQUE DE CALIBRATION**
MAGENBAND
GASTRIC BAND

(30) Priorité: 16.10.2013 FR 1360058
(43) Date de publication de la demande: 24.08.2016
(73) Titulaire: Medical Innovation Developpement, 69570 Dardilly (FR)
(72) Inventeur: CAZENAVE, Ludovic, 69005 Lyon (FR); FRERING, Vincent, 69660 Collonges au Mont d'Or (FR)
(74) Mandataire: Cabinet Plasseraud
(86) Numéro de dépôt international: PCT/FR2014/052611
(87) Numéro de publication internationale: WO 2015/055941

(56) Documents cités:
- EP-A1- 2 468 218
- FR-A1- 2 799 118
- FR-A1- 2 896 148

## Description

### Domaine technique

Le domaine de l'invention est celui des bandes gastriques implantables par voie chirurgicale autour de l'estomac, pour le traitement de l'obésité par gastroplastie FR 2896148 est considéré comme l'état de la technique le plus proche.

### Arrière-plan technologique de l'invention

L'obésité est une menace pour la santé de sujets de plus en plus nombreux. Les traitements de l'obésité sont nombreux tels que les régimes alimentaires, les traitements médicaux, la chirurgie esthétique mais aussi des procédés chirurgicaux. Ainsi, la chirurgie bariatrique est une solution pour soigner les patients en obésité morbide dont l'indice de masse corporelle IMC est supérieure à 40 ou supérieure à 35 avec une co-morbidité. La chirurgie bariatrique consiste à restreindre l'absorption des aliments, diminuant, de fait, l'apport calorique journalier d'un sujet et luttant ainsi contre l'obésité. Elle regroupe un ensemble de techniques qui peuvent être classées en deux types principaux d'interventions :
les premières visent à réduire la capacité gastrique, c'est-à-dire le volume utile de l'estomac, et/ou, à réduire la vitesse de vidange de l'estomac afin d'obtenir un sentiment de satiété plus rapidement. Il s'agit de la gastroplastie par anneau gastrique modulable et de la gastroplastie verticale bandée par agrafage ou gastrectomie longitudinale en manchon appelée aussi "*sleeve gastrectomy"* en anglais;
les secondes, dites mixtes, associent à cette restriction gastrique la création d'un système de dérivation dans le tube digestif afin de diminuer l'absorption des éléments nutritifs par l'intestin : court-circuit gastrique ou by-pass gastrique.

Les secondes techniques d'intervention sont mises en oeuvre principalement après un échec des autres thérapeutiques médicales. Les opérations de gastrectomie longitudinale en manchon ainsi que le by-pass gastrique sont très populaires car elles donnent de bons résultats en termes de perte de poids. Cependant, on a constaté, ces dernières années, une reprise de poids chez les patients ayant subi une opération de by-pass gastrique datant d'une dizaine d'années. Ceci s'explique du fait, qu'au fil du temps, l'estomac se dilate. L'opération de la gastrectomie longitudinale en manchon étant plus récente, la communauté scientifique ne dispose pas encore d'assez de recul pour constater les évolutions et les conséquences de cette opération, mais il est légitime de redouter ce même problème de dilatation.

La nécessité de limiter la dilatation de l'estomac et de calibrer l'estomac pour ces deux opérations semble alors primordiale.

Il est connu, par exemple des demandes de brevet FR 2 799 118, FR 2 799 118, FR 2 981 265, EP 0 611 561, WO 94/27504, WO 2004/019671, d'utiliser des anneaux gastriques gonflables par un liquide physiologique permettant ainsi que contraindre l'estomac et de réduire ainsi son diamètre. Cependant, le gonflage adéquat de l'anneau est une opération délicate et relativement complexe. De plus, il nécessite un boîtier sous-cutané comportant des risques de fuite et de contamination.

Les documents WO 02/096326, FR 2 896 148 et WO 2005/072195 permettent de pallier ces inconvénients en proposant des anneaux gastriques dépourvus de portion gonflable. Cependant, ces dispositifs présentent encore de nombreux inconvénients :
- le diamètre intérieur qu'ils ménagent en position fermée ne peut être ajusté, ce qui engendre des coûts importants car des anneaux de différentes tailles doivent être réalisés,
- ils sont en structure rigide ou semi-rigide et ne sont pas bien supportés par le patient (phénomène d'inclusion),
- ils sont difficiles à poser autour de l'estomac ce qui engendre des risques pour le patient,
- et/ou ils sont dotés de systèmes de fermeture épais et/ou protubérants risquant de blesser l'environnement de l'estomac ou l'estomac lui-même lors de la pose de l'anneau ou une fois l'anneau posé.

### Objectifs de l'invention

C'est à au moins l'un des différents inconvénients susmentionnés qu'entend remédier la présente invention en proposant une bande gastrique non gonflable souple et de préférence préformée pour permettre une pose facilitée.

L'aspect atraumatique, pour l'estomac et son environnement, d'une telle bande gastrique, est particulièrement souhaitable. Ainsi est-il préférable que la bande soit la plus lisse possible sur sa face interne et que les aspérités soient des plus limitées , en particulier au niveau de son système de fermeture, pour éviter les risques de lésions, d'irritation, de perforations de la paroi gastrique ou des organes environnants ou les risques d'inclusion nécessitant une nouvelle intervention chirurgicale.

La présente invention vise à améliorer de manière significative et durable la perte de poids de patients en surpoids ou en obésité morbide.

La présente invention vise également à fournir une bande gastrique à diamètre intérieur ajustable par serrage plus ou moins important de cette dernière autour de l'estomac à entourer, en fonction de la configuration d'implantation et de la morphologie du patient opéré. Cet ajustement est réalisé uniquement lors de la pose de la bande et non ultérieurement.

En résumé, une telle bande a pour but de satisfaire au moins l'un des objectifs énoncés ci-après :
i être simple à mettre en place et à verrouiller autour de la portion d'estomac à entourer et à calibrer.
ii être confortable et sans effet néfaste sur la santé du patient.
iii être biocompatible.
iv être sûre et fiable, notamment indéchirable.
v offrir un verrouillage optimal autour de la portion d'estomac l'estomac à entourer.
vi pouvoir être produite en série industrielle selon des normes drastiques de qualité et au meilleur coût.
vii être au moins en partie radio-opaque.
viii être implantable facilement, de manière stable, non-agressive pour l'estomac et les organes environnants et efficace en terme de calibrage de la portion d'estomac à entourer.

La présente invention vise également à fournir un procédé et un moule pour l'obtention de la bande qui soit viable et simple à mettre en oeuvre à l'échelle industrielle.

La présente invention vise également à fournir une méthode simple, sûre et économique, de traitement de l'obésité, de préférence par calibrage de l'estomac suite à une opération de type gastrectomie longitudinale en manchon ou gastrectomie de type « by-pass », de préférence de type « by-pass », ladite méthode consistant à implanter, par voie chirurgicale, la bande gastrique visée dans les objectifs ci-dessus.

La présente invention vise également à fournir une bande gastrique implantable chirurgicalement, adaptée à un traitement simple, sûr et économique de l'obésité, de préférence par calibrage de l'estomac suite à une opération de type gastrectomie longitudinale en manchon ou gastrectomie de type « by-pass », de préférence de type « by-pass ».

### Brève description de l'invention

Par convention, dans tout le présent exposé, tout singulier désigne aussi un pluriel et réciproquement.

Ces objectifs, parmi d'autres, sont atteints par la présente invention qui concerne une bande gastrique non gonflable comprenant deux zones d'extrémité et une portion intermédiaire à section pleine située entre les deux zones d'extrémité, la bande gastrique présentant une face interne, d'une part, reliée à une face externe de la bande gastrique par deux bords, et, d'autre part, destinée à venir en contact avec la partie de l'estomac ceinte par la bande gastrique, les zones d'extrémité étant équipées respectivement de moyens de fermeture complémentaires aptes à coopérer entre eux pour assurer un verrouillage en position fermée de la bande gastrique, ces moyens de fermeture de la bande gastrique étant choisis parmi au moins un cran et au moins un passant de verrouillage, l'une des zones d'extrémité -ci-après dénommée "zone d'extrémité à cran(s)"-portant au moins un cran, de préférence au moins deux, plus préférentiellement trois crans, et étant destinée à être engagée au travers d'au moins un passant de verrouillage disposé sur la face externe de l'autre zone d'extrémité, jusqu'à une position de fermeture verrouillée dans laquelle au moins un cran est amené au-delà d'au moins un passant de verrouillage,
dans lequel au moins la zone d'extrémité dont la face externe est porteuse d'au moins un passant de verrouillage -ci-après dénommée "zone d'extrémité à passant(s)"- comprend en outre au moins un passant-guide, de préférence plus proche d'une extrémité libre de la zone d'extrémité à passant(s) que le passant de verrouillage, de sorte que lors de la fermeture et du verrouillage de la bande gastrique, le ou les crans passent au travers du passant-guide avant de passer au travers du ou des passants de verrouillage.

La bande gastrique selon la présente invention est un dispositif sensiblement plat, long et étroit, destiné à ceindre une partie de l'estomac. Il s'agit d'une bande de calibration qui, d'une part, donne une dimension voulue à la partie d'estomac disposée en aval ou en amont de la bande de calibration et destinée à recevoir le bol alimentaire, et, qui, d'autre part, empêche, par la contrainte qu'elle impose, la dilatation de cette partie d'estomac réduite et utile au patient pour limiter l'ingestion d'aliments.

Cela évite au patient de subir à nouveau une opération de gastrectomie longitudinale en manchon ou de by-pass gastrique avec tous les risques y afférents.

Cette bande est par ailleurs sûre, fiable, atraumatique pour l'estomac et son environnement, d'un coût raisonnable et susceptible d'être produite à l'échelle industrielle avec les normes de qualité et de productivité requises.

Le passant de verrouillage de la bande a par exemple une forme d'arceau.

Dans le mode de réalisation préféré de l'invention, la bande a un seul passant de verrouillage. Il est situé sur la face externe de la bande et sur la zone d'extrémité à, passant(s) de la bande, de préférence à l'extrémité de celle-ci au niveau de la jonction entre la zone d'extrémité portant le passant de verrouillage et la portion intermédiaire de la bande.

La bande selon l'invention comporte en outre au moins un passant-guide. Il est placé sur la face externe de la bande. Ce passant-guide n'a pas de fonction de verrouillage, il peut faciliter la pose de la bande autour de l'estomac et/ou sa stabilité autour de l'estomac. Lorsque le chirurgien effectue la fermeture de la bande autour de l'estomac, plus précisément lorsque ce dernier amène la zone d'extrémité portant au moins un cran vers la zone d'extrémité portant au moins un passant, lorsque le passant-guide est placé avant le passant de verrouillage, le passant-guide permet d'engager plus facilement la languette ou prolongement de la zone d'extrémité portant au moins un cran vers le passant de verrouillage qui se place dans le même axe longitudinal que le passant-guide. Quelle que soit la position du passant-guide vis-à-vis du passant de verrouillage, le passant-guide permet d'éviter que les bords de la bande en position fermée se décalent de façon angulaire et ne soient plus parallèles. Il permet ainsi de stabiliser la bande en position fermée autour de l'estomac.

Le passant-guide est de préférence en forme d'arceau. Lorsqu'il est situé plus proche de l'extrémité libre de la zone d'extrémité à passant(s) que le passant de verrouillage, il a de préférence une section de passage plus importante, plus large que la section de passage du passant de verrouillage, cette section de passage étant mesurée dans le sens de la largeur de la bande. Lorsque le passant-guide est placé plus loin de l'extrémité libre de la zone d'extrémité à passant(s) que le passant de verrouillage, il peut avoir une section de passage sensiblement identique ou supérieure à celle du passant de verrouillage.

Dans une forme de réalisation préférée de la bande, le passant-guide s'insère de part et d'autre de la bande dans sa largeur et fait toute la largueur de la bande.

Une distance minimale entre le passant-guide et le passant de verrouillage permet d'assurer également la stabilité de la bande une fois fermée et verrouillée.

Cette distance peut être comprise entre 10 mm et 30 mm, de préférence entre 12 mm et 20 mm, par exemple de l'ordre de 15 mm.

De telles dispositions concernant le passant-guide pourraient être mises en oeuvre dans une bande gastrique gonflable.

La bande selon l'invention a une longueur comprise entre 50mm et 90mm, de préférence entre 60 et 80mm, par exemple de l'ordre de 65 et 75mm.

Dans le cas de la calibration de by-pass, la circonférence de la bande est comprise entre 5 cm et 8,5 cm, plus préférentiellement entre 5,5 cm et 7,5 cm, plus préférentiellement encore entre 6 et 7,5 cm.

De façon complémentaire ou indépendante des dispositions concernant le passant-guide, en position fermée de la bande gastrique, la zone d'extrémité à cran(s) et la zone d'extrémité à passant(s) peuvent se chevaucher au moins partiellement et la zone d'extrémité à passant(s) peut être est biseautée dans son épaisseur, de préférence dans la direction longitudinale de la bande.

Selon de telles dispositions, l'invention peut se rapporter à une bande gastrique non gonflable comprenant deux zones d'extrémité et une portion intermédiaire à section pleine située entre les deux zones d'extrémité, la bande gastrique présentant une face interne, d'une part, reliée à une face externe de la bande gastrique par deux bords et, d'autre part, destinée à venir en contact avec la partie de l'estomac ceinte par la bande gastrique,
les zones d'extrémité étant équipées respectivement de moyens de fermeture complémentaires aptes à coopérer entre eux pour assurer un verrouillage en position fermée de la bande gastrique, ces moyens de fermeture de la bande gastrique étant choisis parmi au moins un cran et au moins un passant de verrouillage, l'une des zones d'extrémité -ci-après dénommée "zone d'extrémité à cran(s)"- portant au moins un cran et étant destinée à être engagée au travers d'au moins un passant de verrouillage disposé sur la face externe de l'autre zone d'extrémité jusqu'à une position de fermeture verrouillée dans laquelle au moins un cran est amené au-delà d'au moins un passant de verrouillage,
dans laquelle en position fermée de la bande gastrique, la zone d'extrémité à cran(s) et la zone d'extrémité dont la face externe est porteuse d'au moins un passant de verrouillage - ci-après dénommée "zone d'extrémité à passant(s)"- se chevauchent au moins partiellement, et dans laquelle la zone d'extrémité à passant(s) est biseautée dans son épaisseur, de préférence dans la direction longitudinale de la bande gastrique.

De telles dispositions concernant la zone d'extrémité à passant(s) biseautée pourraient être mises en oeuvre dans une bande gastrique gonflable.

En position fermée, les deux zones d'extrémités de la bande se chevauchent au moins partiellement et sont fixées l'une à l'autre par un système de verrouillage anti-retour "cran/passant". Dans cette position, la face interne (I) de la bande est sensiblement circulaire en vue de côté.

Le chevauchement au moins partiel des deux zones d'extrémité signifie que la zone d'extrémité à cran(s) et la zone d'extrémité à passant(s) se superposent au moins partiellement dans le sens longitudinal de la bande. C'est de préférence, la zone d'extrémité à cran(s) qui recouvre au moins partiellement la zone d'extrémité à passant(s). La superposition n'est pas parfaite nécessairement. Si le chevauchement est complet, alors la zone d'extrémité à passant(s) est recouverte entièrement par la zone d'extrémité à cran(s) et les crans eux-mêmes.

En position fermée de la bande dans laquelle ses zones d'extrémité se chevauchent au moins partiellement, les faces des bords B1, B2 des zones d'extrémité de la bande sont de préférence sensiblement coplanaires.

Suivant une modalité préférée de l'invention, de façon complémentaire ou indépendante des dispositions concernant le passant-guide, la zone d'extrémité à cran(s), est elle aussi biseautée dans son épaisseur, de préférence dans la direction longitudinale de la bande. Plus préférentiellement encore, la zone d'extrémité à cran(s) et la zone d'extrémité à passant(s) présentent respectivement des biseaux complémentaires, en position fermée de la bande gastrique.

Les biseaux sont dits complémentaires car leurs pentes sont inverses, de telle sorte que lorsque le chevauchement des deux zones d'extrémités est complet et que la bande est en position fermée, verrouillée, l'épaisseur de la bande formée par la réunion des deux zones d'extrémité est sensiblement constante en tout point du recouvrement, sans prendre en compte l'épaisseur du ou des passants de verrouillage et/ou du passant-guide de la bande.

De façon préférée, l'extrémité libre de la zone d'extrémité à passant(s) peut être moins épaisse que le reste de la zone d'extrémité à passant(s). Par ailleurs, la zone d'extrémité à cran(s) peut présenter une extrémité libre moins épaisse que le reste de la zone d'extrémité à cran(s).

Dans un mode de réalisation encore plus privilégié, l'épaisseur de la zone d'extrémité à cran(s) et/ou de la zone d'extrémité à passant(s) augmente progressivement de l'extrémité libre jusqu'à la jonction entre ladite zone d'extrémité à cran(s) ou à passant(s) et la portion intermédiaire de la bande.

Par exemple, la face biseautée de la zone d'extrémité à passant(s) est sa face externe de la bande, tandis que la face biseautée de la zone d'extrémité à cran(s) est sa face interne de la bande, l'épaisseur de chaque zone étant croissante à partir de son extrémité libre.

Un tel biseautage permet, d'une part, d'offrir le moins d'aspérité possible, notamment sur la face interne de la bande et, d'autre part, d'avoir une bande la plus fine possible.

La zone d'extrémité à cran(s) peut chevaucher au moins partiellement la zone d'extrémité à passant(s) dont la face interne forme une partie de la face interne de la bande destinée à être en contact avec l'estomac.

Dans une modalité intéressante de la bande selon l'invention, le passant-guide définit un passage pour le(s) cran(s), et ses dimensions, d'une part, sont trop grandes pour permettre un verrouillage anti-retour du (des) cran(s), et, d'autre part, sont supérieures à celles du passage défini par le passant de verrouillage.

Selon une variante, le passant-guide et le passant de verrouillage pourrait ne former qu'un seul passant, avec un passage de dimensions, par exemple de section transversale, décroissantes de l'entrée du passant-guide à la sortie du passant de verrouillage.

Avantageusement, le ou les crans portés par une languette terminale prolongeant la zone d'extrémité à cran(s) et sont disposés sensiblement dans le même plan que celui de la zone d'extrémité à cran(s), le ou les crans étant saillants par rapport aux bords de la languette terminale. Ainsi, le ou les crans portés par la zone d'extrémité à cran(s) sont plus larges que la largeur de la languette dans sa partie terminale.
Le ou les crans ont une forme n'autorisant pas la réouverture de la bande déjà fermée, de telle sorte que lorsque le cran est passé au travers du passant, il offre une résistance contre le passant. Ils ont une forme anti-retour. Ils sont de préférence en forme de sapins ou de flèche, voire en queue d'aronde.

Chaque cran peut présenter une largeur mesurée dans le plan de la bande gastrique, le passant-guide étant réalisé sous la forme d'un arceau présentant une largeur sensiblement égale à la largeur de chaque cran. La zone d'extrémité à cran(s) peut comporter une partie terminale interposée entre une partie comportant chaque cran et la portion intermédiaire, la partie terminale présentant une largeur égale à la largeur de chaque cran.

De préférence, un angle α de biseautage entre la face interne et la face externe de chaque zone d'extrémité à cran(s) (2) ou à passant(s) (3), est compris entre 0,1° et 10°, de préférence entre 0,5° et 8°, et, plus préférentiellement encore entre 1° et 5°.

La mise en place facile et rapide de la bande autour de l'estomac à entourer étant un des avantages recherchés, la bande selon l'invention est de préférence préformée, mieux encore préformée courbe, par exemple en forme de « C ». Cette configuration permet de faciliter la pose de la bande autour de l'estomac pour le chirurgien.

Son extrémité portant un ou des passants de verrouillage comporte en outre, sur sa face externe, au moins un passant-guide, qui, de préférence se situe plus proche de l'extrémité de la zone d'extrémité que le ou les passants de verrouillage. Ainsi, lors de la fermeture et le verrouillage de la bande selon l'invention, lorsque le passant-guide est placé plus proche de l'extrémité libre de la zone d'extrémité à passant(s) que le passant de verrouillage, le ou les crans portés par une zone d'extrémité passent au travers du passant-guide avant de passer au travers du ou des passants de verrouillage portés par l'autre zone d'extrémité. De préférence, le passant-guide a une section de passage, dans la largeur de la bande, plus large que la section de passage du ou des passants de verrouillage.

En position fermée, la face interne de la bande est sensiblement circulaire. Elle est également sans plis, sans invagination, hernie et épouse la portion restante de l'estomac recevant le bol alimentaire de sorte à la calibrer. Elle présente le moins d'aspérité possible de façon à ne pas traumatiser ou blesser l'estomac par des frottements sur la paroi stomacale, des pincements de cette dernière ou autres.

Dans une forme de réalisation préférée, elle est totalement lisse, excepté au niveau de la zone de rencontre entre l'extrémité de la zone d'extrémité portant au moins un passant de verrouillage et la face interne de la bande au niveau de la jonction entre la zone d'extrémité portant les crans et de la portion intermédiaire de la bande. En effet, l'épaisseur du biseau de l'extrémité de la zone d'extrémité portant au moins un passant étant minimale mais ne pouvant être complètement nulle, nécessairement à ce niveau de cette rencontre sus-mentionnée, il y a une légère épaisseur ce qui rend la face interne de la bande en position fermée par totalement lisse mais avec une légère discontinuité à ce niveau.

De préférence, au moins une des arêtes longitudinales de la bande est émoussée ou arrondie de manière à ne pas présenter d'angles saillants susceptibles de blesser le patient.

Cette bande selon l'invention est réalisée dans un matériau souple ou semi rigide de dureté D1 et choisi dans le groupe des élastomères souples, biocompatibles comprenant ou mieux encore constitué par les élastomères silicones ou analogues.
Suivant une caractéristique préférée de l'invention, la dureté D1 de la bande est comprise entre 30 et 80 shoreA, de préférence 50 et 70 shoreA, par exemple de l'ordre de 60 shore A.

En raison de cette implantation précise de la bande sur l'estomac, les dimensions de la bande selon l'invention peuvent être importantes. La bande est donc de préférence étroite, avec de préférence une largeur maximale d'environ 1cm ± 20%.
La longueur de la bande entre ses deux zones d'extrémité peut également avoir une importance car elle détermine la calibration de la poche de l'estomac recevant le bol alimentaire. La bande doit être en contact avec la poche de l'estomac recevant le bol alimentaire sinon elle n'est pas maintenue car elle est non fixée de préférence. Ainsi, la bande ne doit pas être trop longue. Sa longueur doit être comprise entre 50mm et 90mm, de préférence entre 60 et 80mm.

L'épaisseur de la bande variant sur sa longueur. La bande selon l'invention a une épaisseur comprise entre 0,8mm et 2mm.

Il est particulièrement utile que la bande selon l'invention, comprenne au moins une partie radio-opaque (par exemple un insert radio-opaque), de préférence portée par la face externe (E). Ceci permet de la détecter par différents rayonnements, en particulier aux rayons X. Cet insert rend la bande un peu plus épaisse au niveau de celui-ci.

De préférence, les arêtes longitudinales de la face externe sont protubérantes, et, plus préférentiellement encore, sont au moins en partie radio-opaque, notamment aux rayons X.

Pour faciliter le travail du chirurgien, la bande selon l'invention peut comprendre au moins une, de préférence au moins deux saillies de préhension endoscopique, localisée(s) de préférence dans la zone d'extrémité à passant(s), et plus préférentiellement encore, chacune des deux saillies dépassant d'un bord (B1,B2) de la bande, de préférence dans le plan de la bande.

Ces saillies peuvent adopter toutes les formes possibles adaptées à la manipulation sous coelioscopie. De préférence, elles sont arrondies et en forme d'oreillettes avec des bords ne risquant pas de blesser l'estomac ou son environnement.

La bande selon l'invention peut également être caractérisée par des caractéristiques de fabrication. Ainsi, elle peut être avantageusement obtenue par moulage à l'aide d'un moule. Ce dernier présente des caractéristiques qui permettent de définir indirectement la bande selon l'invention. De préférence, le moulage est monobloc.

La présente invention couvre également un procédé d'obtention de la bande susmentionnée qui consiste essentiellement en un moulage, de préférence monobloc ainsi qu'un moule susceptible d'être mis en oeuvre dans ce procédé.

Un autre objet de l'invention est une méthode simple, sûre et économique, de traitement de l'obésité, de préférence par calibrage de l'estomac suite à une opération de type gastrectomie longitudinale en manchon ou gastrectomie de type « by-pass », de préférence de type « by-pass », ladite méthode consistant à implanter, par voie chirurgicale, la bande gastrique susvisée.
Plus précisément, on met en place et on verrouille autour de la portion d'estomac restante recevant le bol alimentaire d'un patient, une bande telle que définie dans le présent exposé, pour réduire éviter la dilation de la portion d'estomac restante.

En pratique, après une gastrectomie longitudinale en manchon ou une gastrectomie by-pass, le chirurgien met en place la bande ouverte selon l'invention autour de la portion d'estomac qui recevra le bol alimentaire ou poche de l'estomac proximal, juste en dessous de l'oesophage. Il ferme cette bande en reliant solidairement ses extrémités l'une à l'autre. De préférence, une fois la bande verrouillée en position fermée autour de l'estomac, la portion de prolongement portant les crans restant au-delà du passant de verrouillage est coupée afin de ne pas blesser l'estomac ou l'environnement de celui-ci, étant entendu que la découpe se fera au-delà du cran afin de ne pas compromettre le verrouillage de la bande. La poche de l'estomac proximal est alors enserrée par la bande qui évite ainsi sa dilatation. Cette opération est délicate et nécessite de la précision car la bande doit être placée de sorte à ne pas compromettre la vascularisation de l'estomac.

De préférence, la bande selon l'invention n'est pas fixée sur l'estomac.

Un autre objet de l'invention est une bande gastrique implantable chirurgicalement, adaptée à un traitement simple, sûr et économique de l'obésité, de préférence par calibrage de l'estomac suite à une opération de type gastrectomie longitudinale en manchon ou gastrectomie de type « by-pass », de préférence de type « by-pass ».

### Description d'un mode de réalisation préféré de l'invention

La description qui suit d'un mode préféré de réalisation de la bande selon l'invention, fera ressortir d'autres de ces caractéristiques remarquables.

Cette description détaillée est faite en référence aux figures annexées dans lesquelles :
- La figure 1 représente une vue en perspective de la bande selon l'invention, non fermée.
- Les figures 2 et 3 représentent des vues de côté de la bande selon l'invention, respectivement en positions ouverte et fermée/verrouillée au niveau du premier cran.
- La figure 3A est une vue de détail de la bande de la figure 3.
- La figure 3B est une variante de la figure 3 fermée/verrouillée au niveau maximum du troisième cran.
- La figure 4 est un détail de la figure 2 (cercle V de la figure 2) montrant en vue de côté la zone d'extrémité à crans, biseautée.
- La figure 5 est un détail de la figure 3 (cercle IV de la figure 3) montrant en vue de côté la zone d'extrémité portant à passant(s), biseautée.
- La figure 6 est une vue de dessus d'une variante de la figure 2.

Comme montré sur les figures annexées, la bande (1) selon l'invention est une bande gastrique destinée à ceindre et calibrer une partie de l'estomac sur lequel elle vient en contact via sa face interne (I).

Cette bande (1) non gonflable, souple, e.g. réalisée par moulage d'un élastomère tel qu'un élastomère silicone, présente une face interne (I) destinée à venir contact avec l'estomac et reliée par deux bords (B1,B2) à une face externe (E). Elle comprend:
- une zone d'extrémité (2), dite "à crans", dont l'extrémité libre (9) présente une extension formée par une languette (10) présentant trois crans (5), respectivement (5.3-5.2-5.1) de la base vers le bout de la languette (10);
- une zone d'extrémité (3) pourvue d'un passant de verrouillage (6) et d'un passant-guide (7) qui s'étendent dans une direction sensiblement perpendiculaire à la face externe de cette zone d'extrémité (3) dite "à passants";
- une portion intermédiaire (4) située entre les deux zones d'extrémité (2) et (3) qui sont deux prolongements de cette portion intermédiaire (4); la face externe (E) de cette portion intermédiaire (4) étant coiffée d'un insert radio-opaque (14).

Les deux zones d'extrémité (2) et (3), la languette (10) et la portion intermédiaire (4) sont à section pleine.

Les trois crans (5) et les passants (6,7) de cette bande (1) de section transversale droite sensiblement rectangulaire, sont des moyens de fermeture (5, 6) complémentaires aptes à coopérer entre eux pour assurer la fermeture et le verrouillage en position fermée de la bande (1) formée par rapprochement des deux zones d'extrémité (2) et (3) l'une vers l'autre.

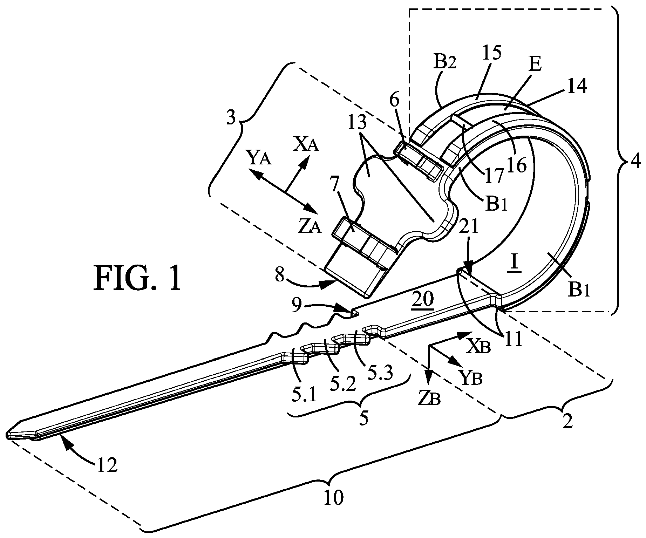

Les trois crans (5.1-5.2-5.3) sont des extensions triangulaires des bords de la languette (10) dans le plan de celle-ci, parallèle au ou dans le plan (x_{B};y_{B}) défini par le repère tridimensionnel (x_{B};y_{B};z_{B}) de la figure 1. Ces trois crans (5.1-5.2-5.3) sont, dans cet exemple de réalisation, de formes et de dimensions identiques. Mais il pourrait en être autrement.

La zone d'extrémité (2) à crans (5) est divisée en deux parties : une partie terminale (20) délimitée par l'extrémité libre (9) et par un épaulement (11) et une partie (21) de liaison s'étendant entre l'épaulement (11) et la jonction entre la zone d'extrémité (2) à crans et la portion intermédiaire (4).

Une surépaisseur (12) de renfort s'étend de la partie terminale (20) de la zone d'extrémité (2) à crans, sur la face externe de la languette (10).

Sur la face externe de la zone d'extrémité (3) à passants, le passant-guide (7) est plus proche de l'extrémité libre (8) de la zone d'extrémité (3), que le passant de verrouillage (6). Le passant de verrouillage (6) est situé au voisinage du sommet de la pente du biseau de la zone d'extrémité (3) à passants, ce sommet étant lui-même sensiblement à la jonction entre la zone d'extrémité (3) à passants et la portion intermédiaire (4).

Par ailleurs, la section de passage du passant-guide (7) de la bande (1) est plus grande que celle du passant de verrouillage (6). Il en va de même pour les largeurs de ces passants selon l'axe (y_{A}) défini par le repère tridimensionnel (x_{A};y_{A};z_{A}) de la figure 1.

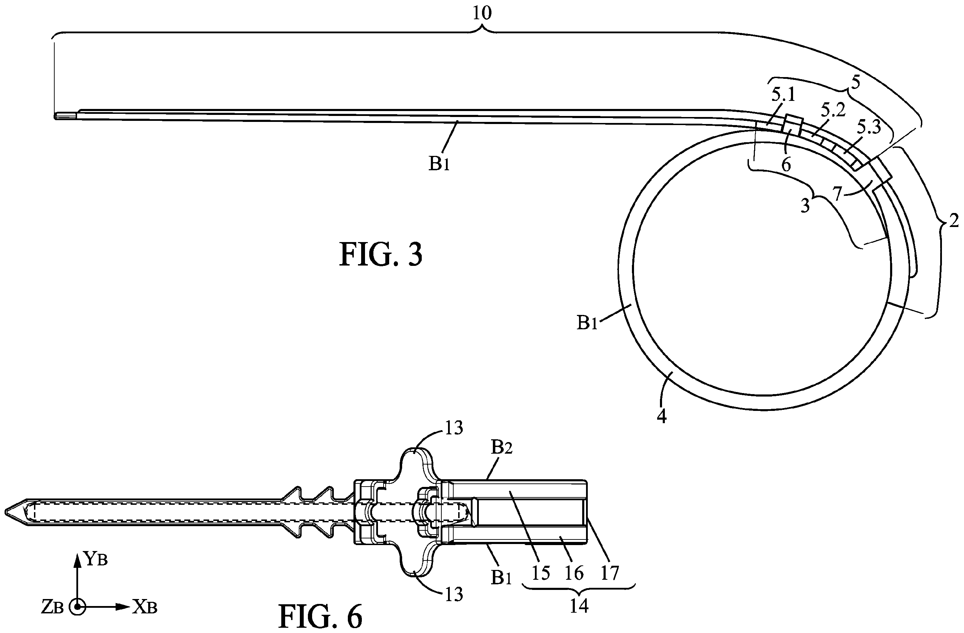

Comme cela ressort de les figures 1 et 6, la bande (1) possède des deux saillies (13) -ou lobes- de préhension endoscopique, dépassant chacune d'un bord (B1, B2) de la zone d'extrémité (3) à passants. Ces lobes 13 se trouvent dans le plan (x_{A},y_{A}) de la largeur de la bande (1).

Dans ce mode préféré de réalisation de la bande (1) selon l'invention, les deux zones d'extrémités (2) et (3) de la bande sont biseautées dans leur épaisseur, selon la direction longitudinale donnée par l'axe (x_{B}),(x_{A}), de sorte que cette épaisseur diminue progressivement de la jonction entre la zone d'extrémité (2),(3) et la portion intermédiaire (4) vers l'extrémité libre (8),(9).

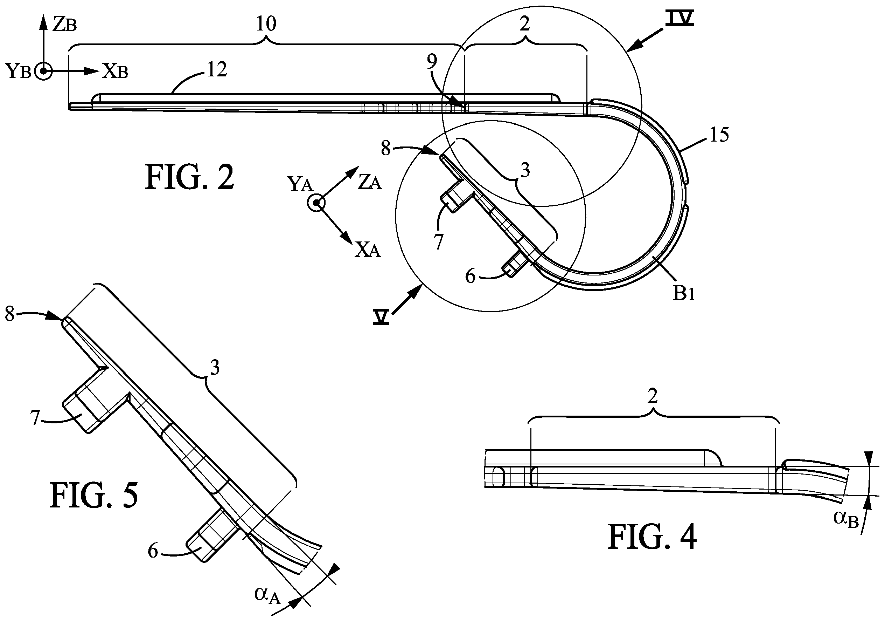

Le biseau de la zone d'extrémité (2) à crans peut être défini par l'angle α_{B} entre sa face interne et sa face externe qui n'est qu'une extension de la face externe (E) de la bande (1) en position fermée et verrouillée de celle-ci. L'angle α_{B} qui apparait sur la figure 4 est compris entre 1 et 5 °, par exemple 4° en l'espèce.

Le biseau de la zone d'extrémité (3) à passants peut être défini par l'angle α_{A} entre sa face externe porteuse des passants (6,7) et sa face interne qui fait partie intégrante de la face interne (I) de la bande (1) en position fermée et verrouillée de celle-ci. L'angle α_{A} qui apparait sur la figure 5 est compris entre 1 et 5 °, par exemple 4° en l'espèce.

De préférence, α_{B} = α_{A} pour le chevauchement soit complémentaire. Ces biseaux servent à améliorer et faciliter la fermeture de la bande autour de l'estomac.

Sur la figure 1, l'insert radio-opaque (14) est formé par deux liserés (15, 16) parallèles, proéminents sur la face (E) et reliées par 3 entretoises (17). Il est constitué par exemple d'un élastomère de même nature ou non que la bande (1).

Comme cela ressort des figures 1 et 2, la bande est préformée de telle sorte que, au repos c'est-à-dire non fermée/verrouillée, elle soit courbe essentiellement dans sa portion intermédiaire (4) -par exemple sur un secteur angulaire compris entre 150 et 270° - 225° en l'espèce-, tandis que la zone d'extrémité (3) à passants, la zone d'extrémité (2) à crans et sa languette crantée prolongeant cette dernière, sont sensiblement rectilignes.
Cette préforme courbe permet de faciliter la fermeture de la bande autour de l'estomac, notamment en limitant les gestes par le chirurgien. De plus, la zone d'extrémité (3) portant au moins un passant (6) est rectiligne et les sections de passage du passant-guide (7) et du passant de verrouillage (6) sont alignées dans le même axe, l'axe longitudinal x_{A} de la bande. Le passage de la languette ou prolongement portant au moins un cran au travers du passant-guide et du passant de verrouillage se fait en un seul mouvement par le chirurgien limitant ainsi les mouvements de ce dernier et le temps d'opération.

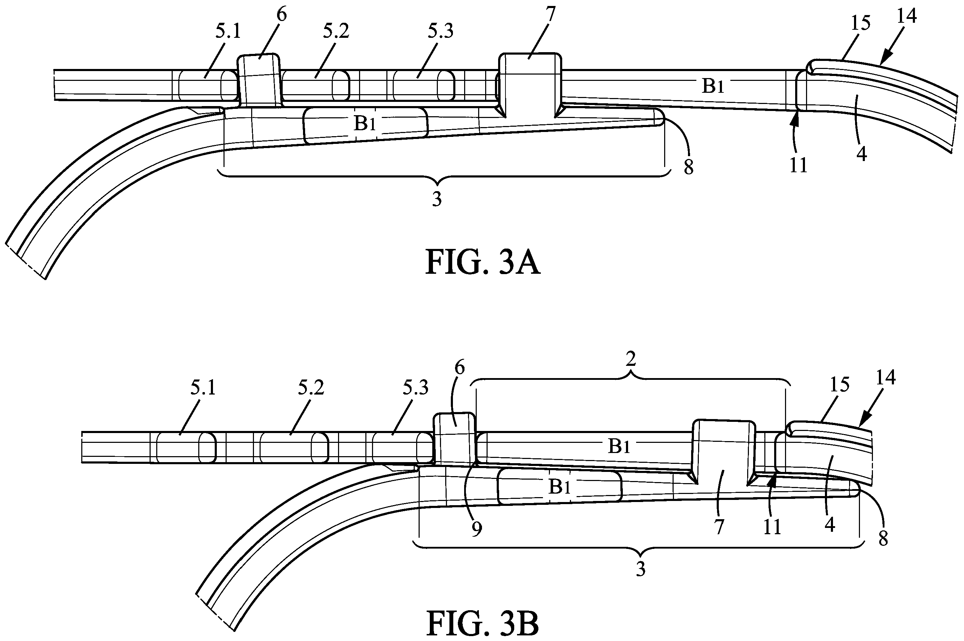

Les figures 3A et 3B montrent respectivement un verrouillage au premier cran (5.1) -le plus proche du bout de la languette (10)- et au troisième cran (5.3) -le plus proche de l'extrémité libre (9) de la zone (2) à crans -.
Dans la position de la figure 3B, le troisième cran (5.3) est passé au travers du passant de verrouillage (6) et sa base vient en appui sur la face du passant (6) tournée vers la portion intermédiaire (4) et l'extrémité libre (9) est en butée contre la face du passant (6) tournée vers le passant-guide (7).

Par ailleurs, l'épaulement (11) entre les parties (20) et (21) de la zone (2) à crans, vient en butée contre la face du passant-guide (7) tournée vers l'extrémité libre (8) de la zone (3) à passants.

Ainsi, la bande peut être fermée/verrouillée de manière simple, sûre, stable et atraumatique, autour de l'estomac. En effet, les déplacements angulaires de la zone d'extrémité portant au moins un cran par rapport à la zone d'extrémité portant au moins un passant sont alors impossibles. Ces deux zones d'extrémité restent bien chevauchantes et leurs bords respectifs restent bien parallèles.

Comme montré sur la figure 3, la bande en position fermée et verrouillée est sensiblement circulaire en vue de côté.

Les figures 3, 3A et 3B montrent que les biseaux des zones d'extrémités respectives (2) et (3) sont en pentes inverses. La zone d'extrémité (2) à crans chevauche donc la zone d'extrémité (3) à passants, de façon complémentaire.

Le recouvrement est plus important dans le verrouillage au cran 5.3 de la figure 3B. Ce chevauchement des biseaux tend à minimiser l'épaisseur (axes z_{A};z_{B} de la figure 1) dans cette zone et limiter, voire supprimer l'aspérité que peut former l'extrémité libre (8) sur la face interne (I) de la bande destinée à être en contact avec l'estomac.

Les arêtes de la bande (1) selon l'invention sont avantageusement émoussées pour ne pas blesser le patient.

Il est prévu conformément, à une caractéristique remarquable de l'invention, que la bande soit monobloc et soit réalisée à partir d'au moins un élastomère silicone de dureté, comprise par exemple entre 30 et 80 shore A, de préférence 50-70 shore A, par exemple de l'ordre de 60 shore A.

La bande (1) selon l'invention peut être mise en place par la voie chirurgicale classique ou par la voie coelioscopique autour de l'estomac et plus précisément autour de la portion de l'estomac recevant le bol alimentaire, dans le cadre de l'opération de « by-pass » autour de la poche de l'estomac proximal. La préforme en "C" et la forme rectiligne des zones d'extrémité et de la languette à crans, de même que les lobes de préhension endoscopique (13) sont autant d'atouts pour faciliter manipulation de cette bande (1) par le chirurgien.

Cette bande (1) ne nécessite pas de fixation à l'estomac, ce qui est un autre avantage décisif pour ne pas alourdir la chirurgie.

## Revendications

1. Bande gastrique (1) non gonflable comprenant deux zones d'extrémité (2, 3) et une portion intermédiaire (4) à section pleine située entre les deux zones d'extrémité (2, 3), la bande gastrique (1) présentant une face interne (I), d'une part, reliée à une face externe (E) de la bande gastrique (1) par deux bords (B1, B2) et, d'autre part, destinée à venir en contact avec la partie de l'estomac ceinte par la bande gastrique (1),
les zones d'extrémité (2, 3) étant équipées respectivement de moyens de fermeture (5, 6) complémentaires aptes à coopérer entre eux pour assurer un verrouillage en position fermée de la bande gastrique (1), ces moyens de fermeture de la bande gastrique (1) étant choisis parmi au moins un cran (5) et au moins un passant de verrouillage (6), l'une des zones d'extrémité (2) -ci-après dénommée "zone d'extrémité (2) à cran(s)"- portant au moins un cran (5) et étant destinée à être engagée au travers d'au moins un passant de verrouillage (6) disposé sur la face externe de l'autre zone d'extrémité (3) jusqu'à une position de fermeture verrouillée dans laquelle au moins un cran (5) est amené au-delà d'au moins un passant de verrouillage (6),
**caractérisée en ce qu'**au moins la zone d'extrémité (3) dont la face externe est porteuse d'au moins un passant de verrouillage (6) -ci-après dénommée "zone d'extrémité (3) à passant(s)"- comprend en outre au moins un passant-guide (7) plus proche d'une extrémité libre de la zone d'extrémité (3) à passant(s) que le passant de verrouillage (6), de sorte que lors de la fermeture et du verrouillage de la bande gastrique (1), le cran (5) passe au travers du passant-guide (7) avant de passer au travers du passant de verrouillage (6).

2. Bande gastrique (1) selon la revendication 1, dans laquelle en position fermée de la bande gastrique (1), la zone d'extrémité (2) à cran(s) et la zone d'extrémité (3) à passant(s) se chevauchent au moins partiellement, et dans laquelle la zone d'extrémité (3) à passant(s) est biseautée dans son épaisseur, de préférence dans la direction longitudinale de la bande gastrique (1).

3. Bande gastrique (1) selon la revendication 2, dans laquelle l'extrémité libre de la zone d'extrémité (3) à passant(s) est moins épaisse que le reste de la zone d'extrémité (3) à passant(s).

4. Bande gastrique (1) selon la revendication 2 ou 3, dans laquelle la zone d'extrémité (2) à cran(s) est biseautée dans son épaisseur, de préférence dans la direction longitudinale de la bande (1).

5. Bande gastrique (1) selon la revendication 4, dans laquelle la zone d'extrémité (2) à cran(s) et la zone d'extrémité (3) à passant(s) présentent respectivement des biseaux complémentaires, en position fermée de la bande gastrique (1).

6. Bande gastrique (1) selon l'une quelconque des revendications 4 et 5, dans laquelle la zone d'extrémité (3) à cran (s) présente une extrémité libre moins épaisse que le reste de la zone d'extrémité à cran(s).

7. Bande gastrique (1) selon l'une quelconque des revendications 2 à 6, dans laquelle en position fermée de la bande gastrique (1), la zone d'extrémité (2) à cran(s) chevauche au moins partiellement la zone d'extrémité (3) à passant(s) dont la face interne forme une partie de la face interne (I) de la bande (1) destinée à être en contact avec l'estomac.

8. Bande gastrique (1) selon l'une quelconque des revendications 2 à 7, dans laquelle un angle α de biseautage entre la face interne et la face externe de chaque zone d'extrémité biseauté est compris entre 0,1° et 10°, de préférence entre 0,5° et 8°, et, plus préférentiellement encore entre 1° et 5°.

9. Bande gastrique (1) selon l'une quelconque des revendications 1 à 8, dans laquelle le passant-guide (7) définit un passage pour le cran, dont les dimensions sont, d'une part, trop grandes pour permettre un verrouillage anti-retour du cran, et, d'autre part, supérieures à celles du passage défini par le passant de verrouillage (6).

10. Bande gastrique (1) selon l'une quelconque des revendications 1 à 9, dans laquelle chaque cran est porté par une languette terminale (10) prolongeant la zone d'extrémité (2) à cran(s) et est disposé sensiblement dans le plan de la bande gastrique (1), chaque cran étant saillant par rapport aux bords de la languette terminale (10).

11. Bande gastrique (1) selon la revendication 10, dans laquelle chaque cran (5) présente une largeur mesurée dans le plan de la bande gastrique (1), le passant-guide (7) est réalisé sous la forme d'un arceau présentant une largeur sensiblement égale à la largeur de chaque cran (5).

12. Bande gastrique (1) selon la revendication 11, dans laquelle la zone d'extrémité (2) à cran(s) comporte une partie terminale (20) interposée entre une partie comportant chaque cran (5) et la portion intermédiaire (4), la partie terminale (20) présentant une largeur égale à la largeur de chaque cran (5).

13. Bande gastrique (1) selon l'une quelconque des revendications 1 à 12, comprenant au moins une partie radio-opaque, de préférence portée par la face externe (E).

14. Bande gastrique (1) selon l'une quelconque des revendications 1 à 13, comprenant au moins une, de préférence au moins deux saillies de préhension endoscopique, localisée(s) de préférence dans la zone d'extrémité (3) à passant(s), et plus préférentiellement encore, chacune des deux saillies dépassant de l'un des bords (B1, B2) de la bande gastrique (1).

## Patentansprüche

1. Nicht aufblasbares Magenband (1), umfassend zwei Endzonen (2, 3) und einen Zwischenabschnitt (4) mit einem durchgehenden Querschnitt, welcher zwischen den beiden Endzonen (2,3) angeordnet ist, wobei das Magenband (1) eine innere Fläche (I) aufweist, welche einerseits mit einer äußeren Fläche (E) des Magenbands (1) durch zwei Ränder (B1, B2) verbunden ist und andererseits dazu vorgesehen ist, in Kontakt mit dem Teil des Magens zu kommen, welcher von dem Magenband (1) umschlossen ist,
wobei die Endzonen (2, 3) jeweils mit komplementären Schließmitteln (5, 6) ausgerüstet sind, welche dazu eingerichtet sind, miteinander zusammenzuwirken, um ein Verriegeln des Magenbands (1) in geschlossener Position sicherzustellen, wobei die Schließmittel des Magenbands (1) aus wenigstens einer Kerbe (5) und wenigstens einer Verriegelungsschlaufe (6) ausgewählt sind, wobei eine der Endzonen (2) - im Folgenden "Endzone (2) mit Kerbe(n)" genannt - wenigstens eine Kerbe (5) trägt und dazu vorgesehen ist, durch wenigstens eine Verriegelungsschlaufe (6) hindurch eingeführt zu werden, welche an der äußeren Fläche der anderen Endzone (3) angeordnet ist, bis zu einer verriegelten Schließposition, in welcher die wenigstens eine Kerbe (5) bis über die wenigstens eine Verriegelungsschlaufe (6) hinaus geführt ist,
**dadurch gekennzeichnet, dass** wenigstens die Endzone (3), an welcher die äußere Fläche die wenigstens eine Verrieglungsschlaufe (6) trägt - im Folgenden "Endzone (3) mit Schlaufe(n)" genannt - ferner wenigstens eine Schlaufenführung (7) näher an einem freien Ende der Endzone (3) mit Schlaufe(n) als die die Verrieglungsschlaufe (6) umfasst, so dass während des Schließens und des Verriegelns des Magenbands (1) die Kerbe (5) durch die Schlaufenführung (7) hindurchtritt, bevor sie durch die Verrieglungsschlaufe (6) hindurchtritt.

2. Magenband (1) nach Anspruch 1, wobei sich in geschlossener Position des Magenbands (1) die Endzone (2) mit Kerbe(n) und die Endzone (3) mit Schlaufe(n) wenigstens teilweise überlappen, und wobei die Endzone (3) mit Schlaufe(n) in ihrer Dicke abgeschrägt ist, vorzugsweise in der longitudinalen Richtung des Magenbands (1).

3. Magenband (1) nach Anspruch 2, wobei das freie Ende der Endzone (3) mit Schlaufe(n) weniger dick als der Rest der Endzone (3) mit Schlaufe(n) ist.

4. Magenband (1) nach Anspruch 2 oder 3, wobei die Endzone (2) mit Kerbe(n) in ihrer Dicke abgeschrägt ist, vorzugsweise in der longitudinalen Richtung des Bands (1).

5. Magenband (1) nach Anspruch 4, wobei die Endzone (2) mit Kerbe(n) und die Endzone (3) mit Schlaufe(n) jeweils komplementäre Abschrägungen in geschlossener Position des Magenbands (1) aufweisen.

6. Magenband (1) nach einem der Ansprüche 4 und 5, wobei die Endzone (3) mit Kerbe(n) ein freies Ende aufweist, welches weniger dick ist als der Rest der Endzone mit Kerbe(n).

7. Magenband (1) nach einem der Ansprüche 2 bis 6, wobei in geschlossener Position des Magenbands (1) die Endzone (2) mit Kerbe(n) wenigstens teilweise die Endzone (3) mit Schlaufe(n) überlappt, deren innere Fläche einen Abschnitt der inneren Fläche (I) des Bands (1) bildet, welcher dazu vorgesehen ist, mit dem Magen in Kontakt zu sein.

8. Magenband (1) nach einem der Ansprüche 2 bis 7, wobei ein Abschrägungswinkel α zwischen der inneren Fläche und der äußeren Fläche von jeder abgeschrägten Endzone zwischen 0,1° und 10° beträgt, vorzugsweise zwischen 0,5° und 8° und weiter vorzugsweise zwischen 1° und 5°.

9. Magenband (1) nach einem der Ansprüche 1 bis 8, wobei die Schlaufenführung (7) einen Durchgang für die Kerbe definiert, dessen Abmessungen einerseits zu groß sind, um eine anti-Umkehr-Verriegelung der Kerbe zu erlauben, und andererseits größer sind als diejenigen des Durchgangs, welcher durch die Verriegelungsschlaufe (6) definiert ist.

10. Magenband (1) nach einem der Ansprüche 1 bis 9, wobei jede Kerbe durch eine Abschlusszunge (10) getragen ist, welche die Endzone (2) mit Kerbe(n) verlängert und im Wesentlichen in der Ebene des Magenbands (1) angeordnet ist, wobei jede Kerbe gegenüber Rändern der Abschlusszunge (10) vorsteht.

11. Magenband (1) nach Anspruch 10, wobei jede Kerbe (5) eine Größe gemessen in der Ebene des Magenbands (1) aufweist, wobei die Schlaufenführung (7) in Form eines Bogens ausgeführt ist, welcher eine Größe aufweist, welche im Wesentlichen gleich der Größe von jeder Kerbe (5) ist.

12. Magenband (1) nach Anspruch 11, wobei die Endzone (2) mit Kerbe(n) einen Abschlussabschnitt (20) umfasst, welcher zwischen einem Teil, welcher jede Kerbe (5) umfasst, und dem Zwischenabschnitt (4) eingefügt ist, wobei der Abschlussabschnitt (20) eine Größe aufweist, welche gleich der Größe von jeder Kerbe (5) ist.

13. Magenband (1) nach einem der Ansprüche 1 bis 12, umfassend wenigstens einen radio-undurchlässigen Teil, vorzugsweise getragen von der äußeren Fläche (E).

14. Magenband (1) nach einem der Ansprüche 1 bis 13, umfassend wenigstens einen, vorzugsweise wenigstens zwei, Vorsprünge zum endoskopischen Greifen, welche(r) vorzugsweise in der Endzone (3) mit Schlaufe(n) angeordnet ist/sind, und wobei weiter vorzugsweise jeder der beiden Vorsprünge über einen der Ränder (B1, B2) des Magenbands (1) übersteht.

## Claims

1. Non-inflatable gastric band (1) comprising two end areas (2, 3) and an intermediate portion (4) with a solid cross section situated between the two end areas (2, 3), the gastric band (1) having an inner surface (I), on the one hand, linked to an outer surface (E) of the gastric band (1) by two edges (B1, B2) and, on the other hand, intended to come into contact with the part of the stomach encircled by the gastric band (1),
the end areas (2, 3) being equipped respectively with complementary closing means (5, 6) capable of cooperating with each other in order to ensure a locking in the closed position of the gastric band (1), these closing means of the gastric band (1) being chosen from at least one notch (5) and at least one locking loop (6), one of the end areas (2) - hereafter referred to as "end area (2) with notch(es)" - bearing at least one notch (5) and being intended to be inserted through at least one locking loop (6) arranged on the outer surface of the other end area (3) up to a locked closed position in which at least one notch (5) is moved beyond at least one locking loop (6),
**characterized in that** at least the end area (3) the outer surface of which bears at least one locking loop (6) - hereafter referred to as "end area (3) with loop(s)" - also comprises at least one guide loop (7), preferably closer to a free end of the end area (3) with loop(s) than the locking loop (6), so that during the closing and locking of the gastric band (1), the notch (5) passes through the guide loop (7) before passing through the locking loop (6).

2. Gastric band (1) according to claim 1 in which, in the closed position of the gastric band (1), the end area (2) with notch(es) and the end area (3) with loop(s) overlap at least partially, and in which the end area (3) with loop(s) is bevelled in its thickness, preferably in the longitudinal direction of the gastric band (1).

3. Gastric band (1) according to claim 2, in which the free end of the end area (3) with loop(s) is thinner than the remainder of the end area (3) with loop(s).

4. Gastric band (1) according to claim 2 or 3, in which the end area (2) with notch(es) is bevelled in its thickness, preferably in the longitudinal direction of the band (1).

5. Gastric band (1) according to claim 4, in which the end area (2) with notch(es) and the end area (3) with loop(s) respectively have complementary bevels in the closed position of the gastric band (1).

6. Gastric band (1) according to any one of claims 4 and 5, in which the end area (3) with notch(es) has a free end thinner than the remainder of the end area with notch(es).

7. Gastric band (1) according to any one of claims 2 to 6 in which, in the closed position of the gastric band (1), the end area (2) with notch(es) at least partially overlaps the end area (3) with loop(s) the inner surface of which forms a part of the inner surface (I) of the band (1) intended to be in contact with the stomach.

8. Gastric band (1) according to any one of claims 2 to 7, in which an angle α of the bevel between the inner surface and the outer surface of each bevelled end area is comprised between 0.1° and 10°, preferably between 0.5° and 8°, and, even more preferentially between 1° and 5°.

9. Gastric band (1) according to any one of claims 1 to 8, in which the guide loop (7) defines a passage for the notch, the dimensions of which are, on the one hand, too large to allow an anti-reverse locking of the notch, and, on the other hand, greater than those of the passage defined by the locking loop (6).

10. Gastric band (1) according to any one of claims 1 to 9, in which each notch is borne by a terminal tongue (10) extending the end area (2) with notch(es) and is arranged substantially in the plane of the gastric band (1), each notch projecting with respect to the edges of the terminal tongue (10).

11. Gastric band (1) according to claim 10, in which each notch (5) has a width measured in the plane of the gastric band (1), the guide loop (7) is produced in the form of an arch having a width substantially equal to the width of each notch (5).

12. Gastric band (1) according to claim 11, in which the end area (2) with notch(es) comprises a terminal part (20) interposed between a part comprising each notch (5) and the intermediate portion (4), the terminal part (20) having a width equal to the width of each notch (5).

13. Gastric band (1) according to any one of claims 1 to 12, comprising at least one radio-opaque part, preferably borne by the outer surface (E).

14. Gastric band (1) according to any one of claims 1 to 13, comprising at least one, preferably at least two projections for endoscopic handling, preferably located at the end area (3) with loop(s), and even more preferentially, each of the two projections extending from one of the edges (B1, B2) of the gastric band (1).
